(19) Europäisches Patentamt
European Patent Office
Office européen des brevets

(11) Publication number: **0 507 008 B1**

# (12) EUROPEAN PATENT SPECIFICATION

(45) Date of publication of patent specification: **07.06.95**

(51) Int. Cl.$^6$: **C08F 20/60**, C07C 381/00, G03F 7/038, C08F 12/30

(21) Application number: **91200512.1**

(22) Date of filing: **08.03.91**

## (54) Lithographic printing plate based on a resin comprising aryldiazosulfonates.

(43) Date of publication of application:
**07.10.92 Bulletin 92/41**

(45) Publication of the grant of the patent:
**07.06.95 Bulletin 95/23**

(84) Designated Contracting States:
**BE DE FR GB NL**

(56) References cited:
**EP-A- 0 339 393**
**DE-A- 1 959 052**

**PATENT ABSTRACTS OF JAPAN vol. 8, no. 109 (P-275)(1546) 22 May 1984 & JP- A-59 017 549**

(73) Proprietor: **AGFA-GEVAERT naamloze vennootschap**
**Septestraat 27**
**B-2640 Mortsel (BE)**

(72) Inventor: **Nuyken, Oskar**
**Ignaz-Güntherstrasse 12**
**D 8000 München 81 (DE)**
Inventor: **Dauth, Jochen, Walter**
**Peter Rosegger Weg 13**
**D 8580 Bayreuth (DE)**
Inventor: **Vermeersch, Joan Triphon**
**Kernlaan 12**
**B 9800 Deinze (BE)**

EP 0 507 008 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

1. Field of the invention.

The present invention relates to an aryldiazosulfonate containing resin, a method for preparing said resin, an imaging element comprising said resin and a method for preparing a lithographic printing plate using said imaging element.

2. Background of the invention.

Lithography is the process of printing from specially prepared surfaces, some areas of which are capable of accepting lithographic ink, whereas other areas, when moistened with water, will not accept the ink. The areas which accept ink form the printing image areas and the ink-rejecting areas form the background areas.

In the art of photolithography, a photographic material is made image-wise receptive to oily or greasy inks in the photo-exposed (negative-working) or in the non-exposed areas (positive-working) on a hydrophilic background.

In the production of common lithographic printing plates, also called surface litho plates or planographic printing plates, a support that has affinity to water or obtains such affinity by chemical treatment is coated with a thin layer of a photosensitive composition. Coatings for that purpose include light-sensitive polymer layers containing diazo compounds, dichromate-sensitized hydrophilic colloids and a large variety of synthetic photopolymers.

Particularly diazo-sensitized systems are widely used. These systems have been extensively reviewed by Kosar J. in "Light-Sensitive Systems", Wiley, New York, 1965, Chapter 7.

A generally used negative-working diazo-sensitized system is based on the capability of diazo compounds to harden a polymer when exposed to ultraviolet and blue radiation. Diazo compounds which have been used for the preparation of lithographic printing plates based on their hardening properties are e.g. diazonium salts whose photolysis products can harden polymers (natural colloids or synthetic resins) directly and diazonium polymers. Although polymers containing diazonium groups have a large structure they remain water soluble owing to the presence of the ionic diazonium groups. When these groups are destroyed by exposure to light an insoluble resin is formed. Particularly useful diazonium polymers are the condensation products of a carbonyl compound, e.g. an aldehyde, such as formaldehyde, with a diazonium salt of e.g. a p-aminodiphenylamine. These condensation products are usually designated diazo resins. In these systems a polymeric binder is optionally added to the diazo resin coating.

Upon image-wise exposure of an imaging element comprising a light-sensitive diazo resin layer the exposed image areas become water insoluble and the unexposed areas remain water soluble. The plate is then developed to remove the diazo resin in the unexposed areas.

Altough processing with plain water would be an ecological advantage it is often not possible due to diazo resin remaining in the non-exposed areas. Therefore aqueous solutions containing agents to facilate the removal of the diazo resin in the non-exposed areas are added e.g. phosphates. In addition to the processing with water processing can also be performed using an organic solvent or mixture thereof with water.

Diazo resins based on polymers containing aryldiazosulfonate units where the sulfonate group is directly attached to the diazo function, are known and desribed in EP 339393. These polymers containing aryldiazosulfonate units are highly water soluble and can be processed with plain water. However the aryldiazosulfonates disclosed in EP 339393 have the disadvantage that they are only sensitive to UV-light of short wavelengths typically around 300nm while commonly employed light sources in the art of photolithography comprise wavelengths above 320nm.

3. Summary of the invention.

It is thus an object of the present invention to provide aryldiazosulfonate based resins that are highly water soluble and that are sensitive to wavelengths above 320nm.

It is a second object of the present invention to provide a method for producing a lithographic printing plate based on an aryldiazosulfonate based resin.

Still further objects of the present invention will become clear from the description hereinafter.

According to the present invention polymerizable aryldiazosulfonates of the general formula (I) are provided:

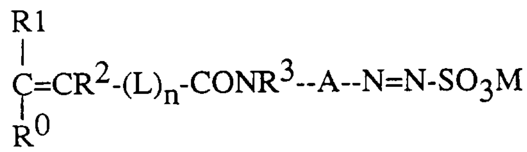

(I)

wherein each of $R^0$, $R^1$ and $R^2$ which may be the same or different, represents hydrogen or a methyl group, $R^3$ represents hydrogen or an alkyl group, A represents an aromatic divalent group which may be substituted or not, including a fused aromatic divalent group e.g. phenylene, naphthylene, methoxyphenylene, dimethoxyphenylene, ethoxyphenylene, methoxynaphthylene, ethoxynaphthylene, methylphenylene, methylmethoxyphenylene, methylnaphthylene, N,N'-dimethylaminophenylene, N,N'-diethylaminonaphthylene etc., L represents a organic divalent group e.g. alkylene, alkoxyalkylene, alkyleneoxy, arylene, substituted arylene, O, S, NR with R being a hydrogen or lower alkyl group, M represents hydrogen, $NH_4$, Al, Zn, Cu, an alkali metal or an alkaline earth metal and n may be 0 or 1.

According to the present invention there is also provided a resin comprising a polymer comprising aryldiazosulfonate units according to general formula (II):

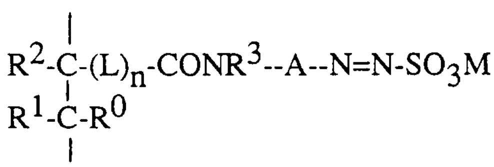

(II)

wherein $R^0$, $R^1$, $R^2$, $R^3$, A, L, n and M have the same meaning as defined in general formula (I).

According to the present invention there is also provided an imaging element comprising a layer containing a polymer having aryldiazosulfonate units corresponding to general formula (II).

According to the present invention there is also provided a method for obtaining a lithographic printing plate comprising the steps of (i) image-wise exposing an imaging element comprising on a support a layer of a polymer comprising aryldiazosulfonate units corresponding to general formula (II) and (ii) developing said image-wise exposed imaging element.

4. Detailed description of the invention.

It was found that the aryldiazosulfonate based resins of the present invention show a maximum spectral sensitivity at a wavelength at or above 320nm. The aryldiazosulfonate based resins of the present invention therefor offer the advantage of higher sensitivity, with respect to the aryldiazosulfonate based resins described in EP 339 393, towards commonly applied U.V.-light sources for the exposure of imaging elements in the production of lithographic printing plates.

Examples of units corresponding to general formula (II) are shown in table 1.

Table 1

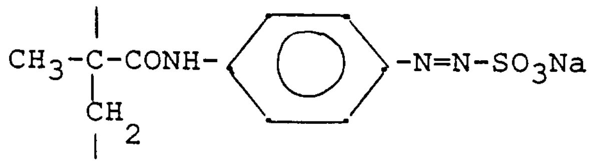

1

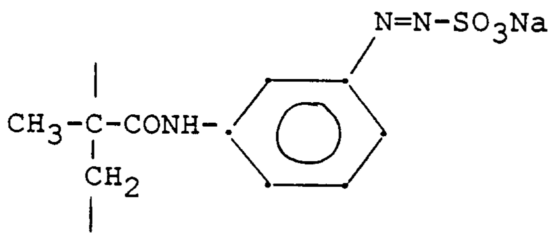

2

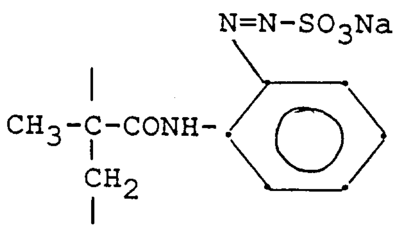

3

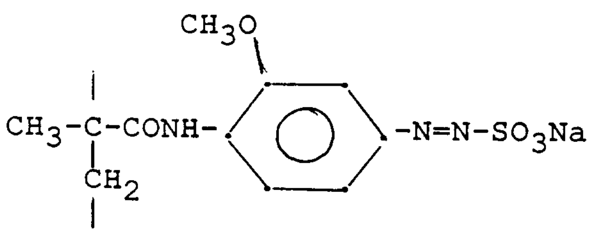

4

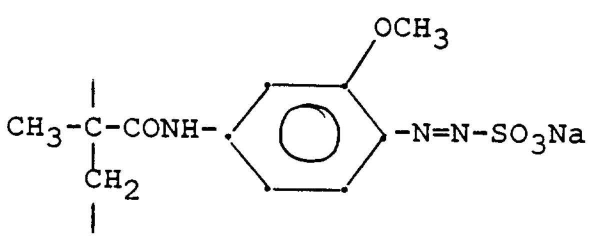

5

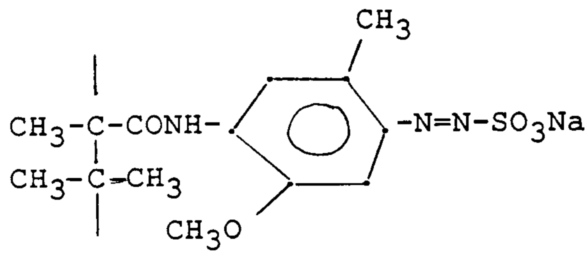

6

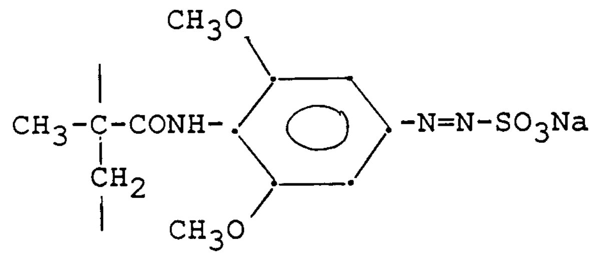

7

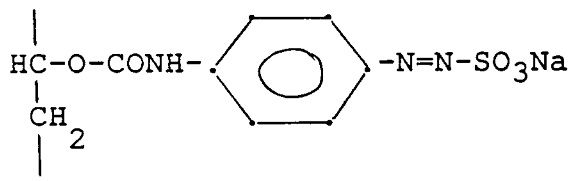

8

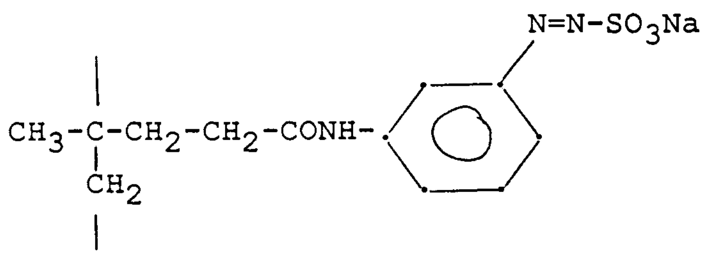

9

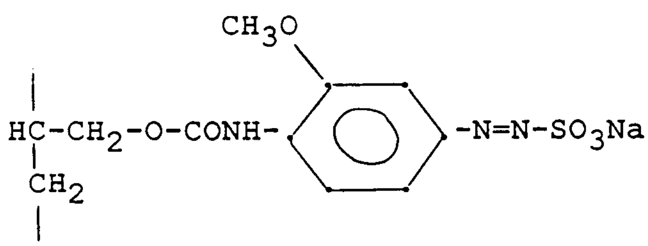

10

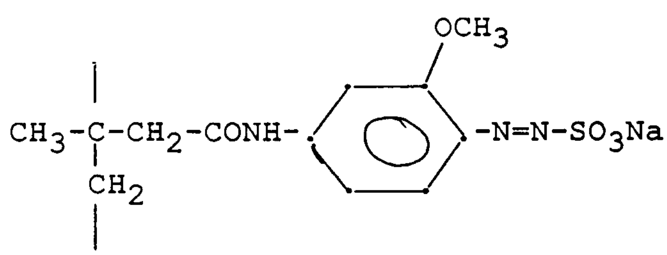

11

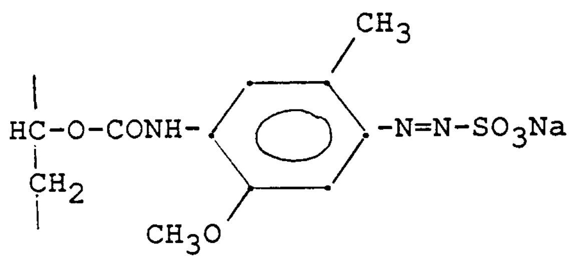

12

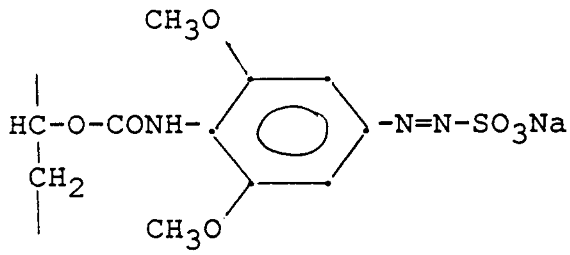

13

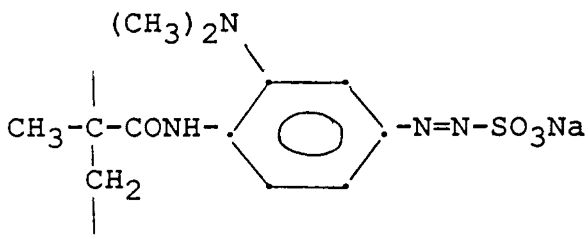

14

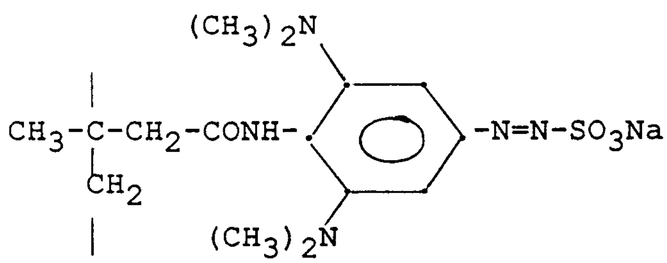

15

According to a first embodiment of the present invention the polymers of the present invention comprising aryldiazosulfonate units corresponding to general formula (II) can be prepared by the radical polymerization method of the corresponding monomer corresponding to general formula (I). Examples of monomers corresponding to general formula (I) are shown in table 2.

Table 2

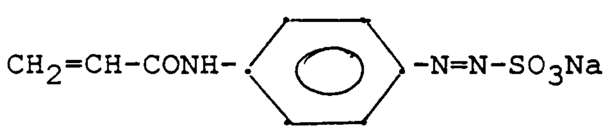

1

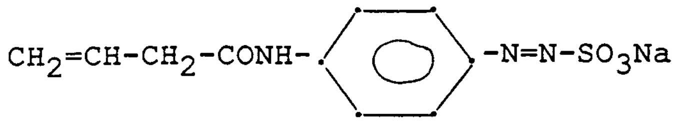

2

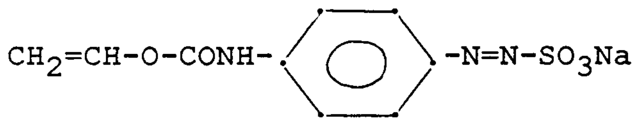

3

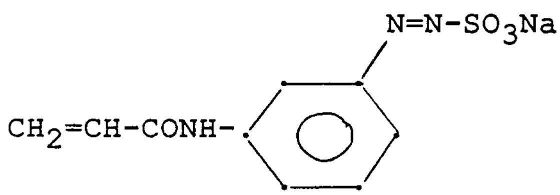

4

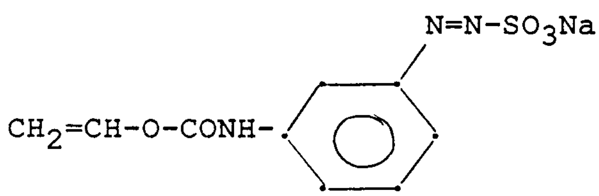

5

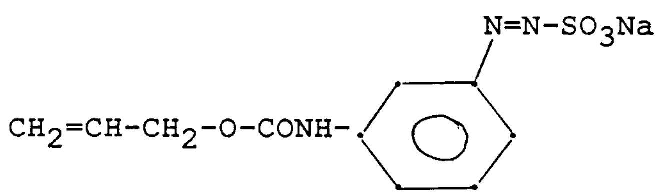

6

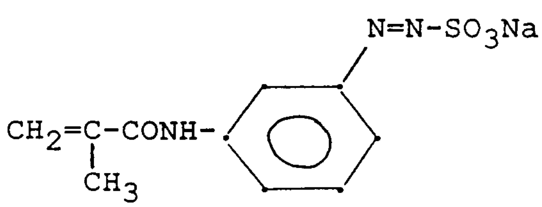

7

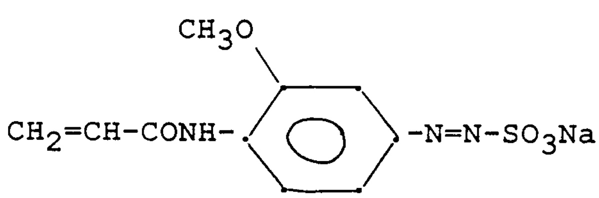

8

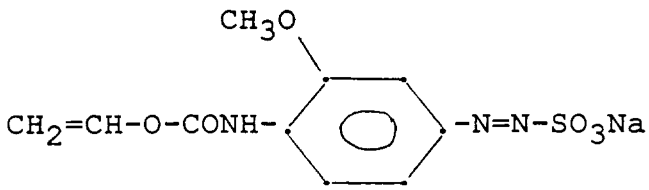

9

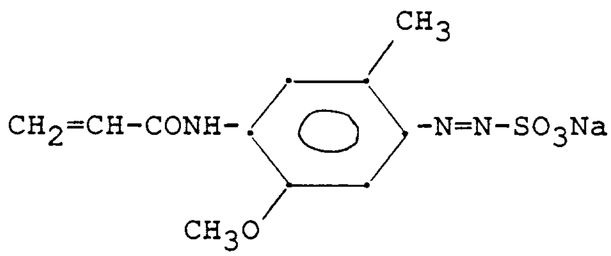

10

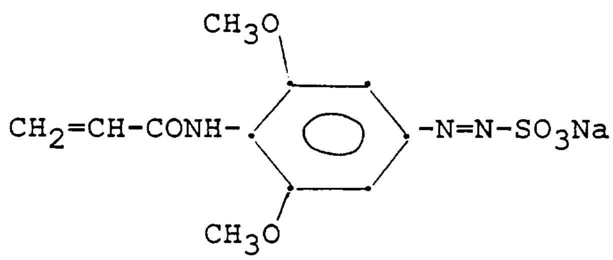

11

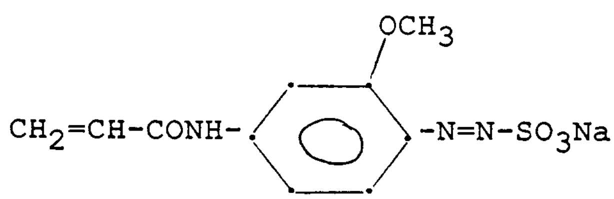

12

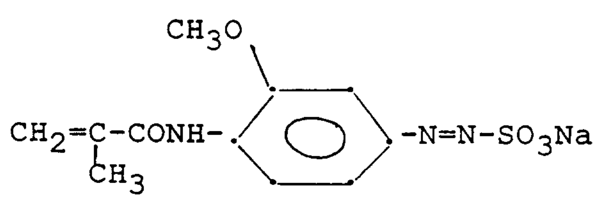

13

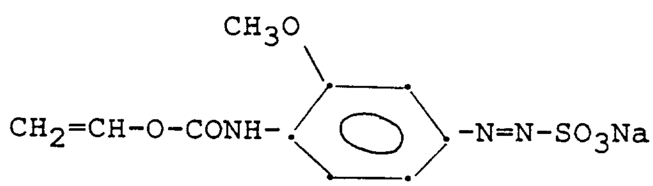

14

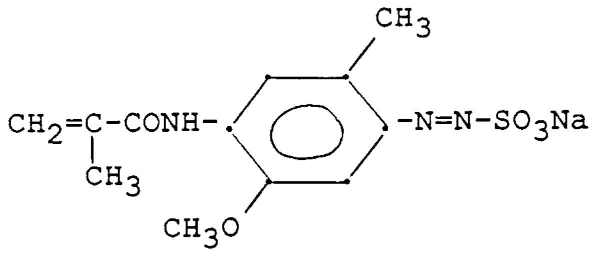

15

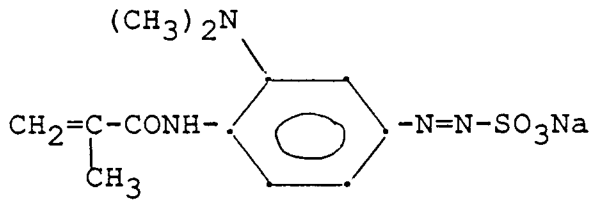

16

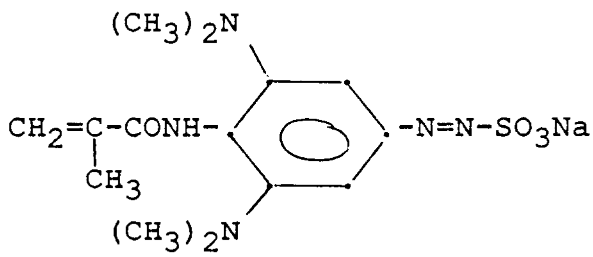

17

The monomers shown in the above table 2 may be prepared by reacting the appropriate amine substituted aryldiazosulfonate, e.g. the compounds shown in table 3, with the acid halide or acid derivative of an ethylenic unsaturated polymerizable group. The aryldiazosulfonate compounds can be prepared similar to the procedure described in EP 339393. A typical example of the preparation of monomers corresponding to general formula I is given in example 2 below.

The monomers of the present invention corresponding to general formula (I) may be homopolymerized or copolymerized with one or more monomers e.g. esters of methacrylic acid e.g. methyl methacrylate, ethyl methacrylate, esters of acrylic acid e.g. methyl acrylate, ethyl acrylate, methacrylic acid, acrylic acid, acrylamide, methacrylamide, acrylonitrile, vinyl acetate, vinyl chloride, vinylidene chloride, styrene, alpha-methyl styrene, etc. or with another monomer corresponding to general formula (I) in the presence or absence of one or more vinyl monomers.

When the monomers corresponding to general formula (I) are copolymerized with one or more vinyl monomers the amount of monomers corresponding to general formula (I) contained in the final copolymer should be such that the copolymer is readily soluble in water. The copolymer is readily soluble in water when not more than 3% by weight remains insoluble at 45 °C. Preferably the copolymer contains 10mol% to 50mol% of at least one aryldiazosulfonate monomer corresponding to general formula (II).

According to the most preferred embodiment of the present invention the polymers containing aryldiazosulfonate units corresponding to general formula (II) may be prepared by reaction of a polymer containing acid groups or acid halide groups with a appropriate amine substituted aryldiazosulfonate. Examples of such amine substituted aryldiazosulfonates suitable for use in accordance with the present invention are shown in table 3.

Table 3

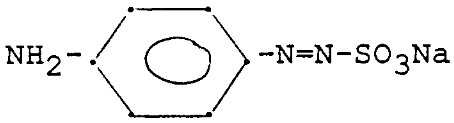

1

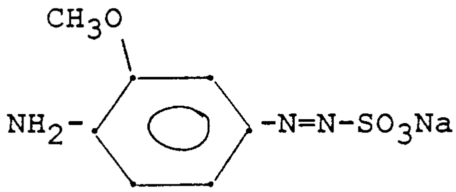

2

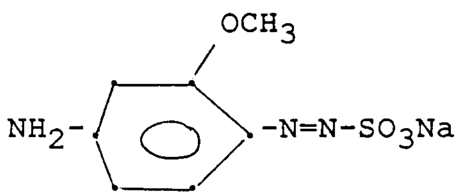

3

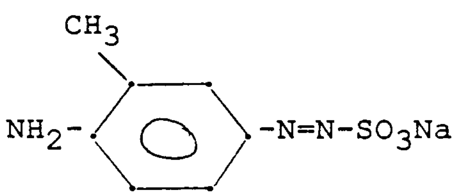

4

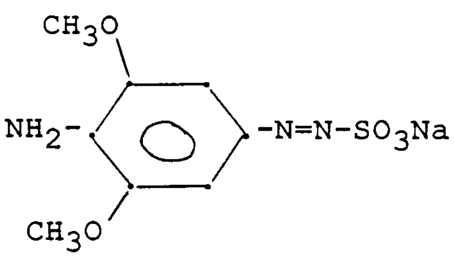

5

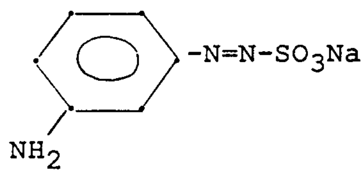

6

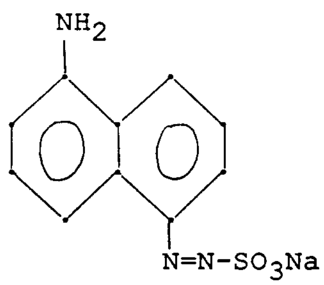

7

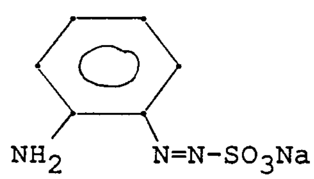

8

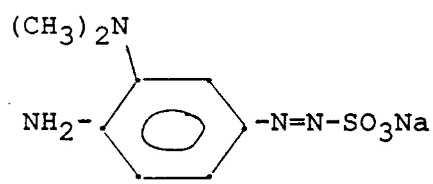

9

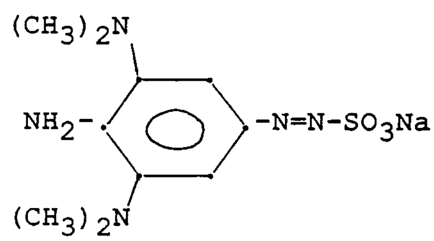

10

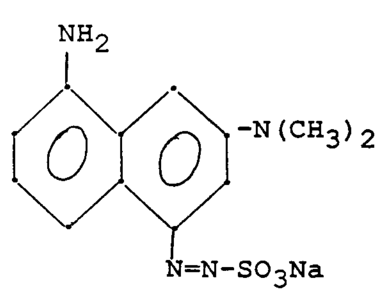

11

Suitable polymers for use in accordance with the second embodiment of the present invention are e.g. homopolymers or copolymers of acryloyl chloride or methacryloyl chloride, homopolymers or copolymers of acrylic acid or methacrylic acid, homopolymers or copolymers of monomers shown in table 4, etc.. Preferably used polymers in accordance with the present invention are homopolymers or copolymers of (meth)acryloyl chloride.

Table 4

| | |
|---|---|
| $CH_2=CH-O-COCl$ | 1 |
| $CH_2=CH-CH_2-COCl$ | 2 |
| $CH_2=CH-CH_2-CH_2-O-COCl$ | 3 |
| 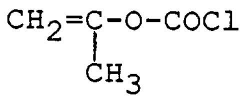 | 4 |
| 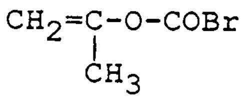 | 5 |
| 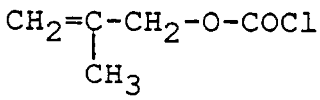 | 6 |
| 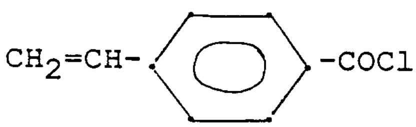 | 7 |

The method according to the second embodiment of the present invention offers the advantage that the molecular weight can be controlled in advance. The most important advantage of this method is however the possibility to prepare aryldiazosulfonate copolymers containing strongly hydrophobic comonomers. These copolymers cannot be prepared by the radical copolymerization method because such method requires that all the monomers are solubilised in a common solvent which is impossible for strongly hydrophobic monomers in combination with aryldiazosulfonate monomers corresponding to general formula (I) which are essentially hydrophilic in nature. Aryldiazosulfonate copolymers comprising strongly hydrophobic comonomers are advantageous for use in the manufacturing of lithographic printing plates because the differentiation between the oleophilic and oleophobic areas of the plate can be enhanced with these copolymers.

The above described method can also be employed to prepare aryldiazosulfonate polymers containing an ester group instead of the amide group bound to the arylene (symbol A) in general formula (II). Instead of compounds corresponding to general formula (III) aryldiazosulfonate compounds having an OH function instead of the amine function of the compounds corresponding to general formula (III) are then allowed to react with a polymer containing acid or carboxy halide to form the ester link to the polymer backbone. These polymers are disclosed in EP 339393 but have the disadvantage of having a maximum spectral sensitivity of about 290nm thus making them less suitable for exposure with commonly employed light source in the art of lithography.

The aryldiazosulfonate containing polymers of the present invention are especially suitable for use in the production of lithographic printing plates. According to the present invention there is thus provided an imaging element comprising a support coated with a layer containing a polymer having aryldiazosulfonate units corresponding to general formula (II).

Several types of supports can be used for the manufacturing of the imaging element of the present invention. Suitable supports for use in accordance with the present invention are metal supports like Al or Zn, polyester film supports and paper bases. These supports, if not sufficiently hydrophilic by themselves, are first coated with a hydrophilic layer to form the hydrophilic background of the printing plate and a top layer containing the diazo compound is then applied (see for example DE-P-1900469, DE-P-2030634 and US-P-3971660). A particularly suitable hydrophilic layer is a layer of polyvinyl alcohol hardened with tetraalkyl orthosilicate e.g. tetramethylorthosilicate or tetraetylorthosilicate containing $TiO_2$ as disclosed in e.g. US-P-3971660.

A support preferably used with the present invention is aluminium. Suitable aluminium supports for use in accordance with the present invention are aluminium foils made of pure aluminium or of an aluminium alloy, the aluminium content of which is at least 95%. A useful alloy is e.g. one comprising 99.55% by weight of Al, 0.29% of Fe, 0.10% of Si, 0.004% of Cu, 0.002% of Mn, 0.02% of Ti, and 0.03% of Zn. The thickness of the foil usually ranges from about 0.13 to about 0.50 mm.

The preparation of aluminium or aluminium alloy foils for lithographic offset printing comprises the following steps : graining, anodizing, and optionally sealing of the foil.

Graining and anodization of the foil are necessary to obtain a lithographic printing plate that allows to produce high-quality prints in accordance with the present invention. Sealing is not necessary but may still improve the printing results.

Graining of the aluminium surface can be carried out mechanically or electrolytically in any known way. The roughness produced by the graining is measured as a centre line average value expressed in µm and preferably varies from about 0.2 to about 1.5 µm.

The anodization of the aluminium foil can be performed in electrolytes e.g. chromic acid, oxalic acid, sodium carbonate, sodium hydroxide, and mixtures thereof. Preferably, the anodization of the aluminium is performed in dilute aqueous sulphuric acid medium until the desired thickness of the anodization layer is reached. The aluminium foil may be anodized on both sides. The thickness of the anodization layer is most accurately measured by making a micrographic cut but can be determined likewise by dissolving the anodized layer and weighing the plate before dissolution treatment and subsequent thereto. Good results are obtained with an anodization layer thickness of about 0.4 to about 2.0 µm. To promote the image sharpness and, as a consequence thereof, the sharpness of the final printed copy, the anodization layer may be coloured in the mass with an antihalation dye or pigment e.g. as described in JA-A 58-14797. The dye or pigment or a combination of dyes or pigments used for such colouring in the mass are chosen such that they prevent or reduce halation in the layer comprising the polymer containing aryldiazosulfonates.

After the anodizing step the anodic surface may be sealed. Sealing of the pores of the aluminium oxide layer formed by anodization is a technique known to those skilled in the art of aluminium anodization. This technique has been described in e.g. the "Belgisch-Nederlands tijdschrift voor Oppervlaktetechnieken van materialen", 24ste jaargang/januari 1980, under the title "Sealing-kwaliteit en sealing-controle van geanodiseerd Aluminium". Different types of sealing of the porous anodized aluminium surface exist. An advantageous sealing method is the hydration-sealing method, according to which the pores are closed or partially closed through water-acceptance so that hydrated needle-like aluminium oxide crystals (böhmite) are formed. The anodic surface of the aluminium foil can thus be rinsed with water at 70-100°C or with steam. The hot sealing water may comprise additives e.g. nickel salts to improve the sealing effect. The sealing can also be performed by treatment of the anodic surface with an aqueous solution comprising phosphate ions or silicates. Thanks to the sealing treatment the anodic layer is rendered substantially non-porous so that longer press runs can be made with the printing plate obtained. As a result of the sealing the occurrence of fog in the non-printing areas of the printing plate is avoided substantially.

The graining, anodizing, and sealing of the aluminium foil can be performed as described in e.g. US-A 3,861,917 and in the documents referred to therein.

The imaging element of the present invention advantageously contains water-soluble dyes e.g. rhodamines, sudan blue, methylen blue, eosin or trifenylmethane dyes e.g. as crystal violet, victoria pure blue, malachite green, methylviolet and fuchsin. The imaging element preferably also contains dye pigments which are essentially water insoluble. These dyes or dye pigments may be present in the light sensitive layer containing the aryldiazosulfonate polymer of the present invention or in another layer comprised on the support of the imaging element e.g. the hydrophilic layer of polyvinyl alcohol hardened with tetraalkyl orthosilicate described above.

The thickness of the light-sensitive layer in the material of this invention may vary in the range of 0.1 to 10 µm and is preferably between 0.5 and 2.5 µm.

The coating composition of the invention is coated using any conventional coating method.

According to the method of the present invention for producing a lithographic printing plate the above described imaging element of the invention is image-wise exposed. The exposure of the imaging element advantageously proceeds with ultraviolet light optionally in combination with blue light in the wavelength range of 250 to 500 nm. Useful exposure sources are high or medium pressure halogen mercury vapour lamps, e.g. of 1000 W.

Since most lithography is done by the offset process, the imaging element is exposed in such a way that the image obtained thereon is right reading. The exposure may be an exposure using optics or a contact exposure.

The aryldiazosulfonate resin is converted upon exposure from water soluble to water insoluble (due to the destruction of the diazosulfonate groups) and additionally the photolysis products of the diazo may induce an advancement in the level of crosslinking of the polymeric binder or diazo containing polymer, thereby selectively converting the surface, in an image pattern, from water soluble to water insoluble. The unexposed areas remain unchanged, i.e. water soluble.

When mounted on a printing press the printing plate is first wiped with an aqueous fountain solution. To prevent this fountain solution from being contaminated by residual non-exposed diazo which are water soluble, the exposed imaging element should first be developed to remove the unexposed polymer containing aryldiazosulfonates before mounting it on a printing press. This development can be achieved by washing the imaging element with plain water.

The following examples illustrate the present invention.

The percentages given are by weight unless otherwise stated.

EXAMPLE 1

Preparation of the sodium salt of p-amino-phenyldiazosulfonate (compound 1 of table 3).

The following 3 solutions were prepared and cooled to -5 ° C.

Solution A: 2.7g of 1,4-diaminobenzene was dissolved in 30ml of an aqueous solution containing 10% HCl.

Solution B: 1.7g of $NaNO_2$ was dissolved in 30ml of water.

Solution C: 6g of $Na_2SO_3$ and 10g of $Na_2CO_3$ were dissolved in 100ml of water.

Solution B was slowly added to solution A while stirring. The color of solution A changed to dark brown. When solution B was completely added to solution A the resulting mixture was added to solution C in 10 min. while stirring. The resulting reaction mixture was filtered, evaporated to a volume of 100ml and subsequently kept in a refrigirator. Afier 12 hours fine yellow cristals of the sodium salt of p-amino-phenyldiazosulfonate were obtained.

Preparation of the aryldiazosulfonate containing copolymer.

To 40ml distilled, dry tetrahydrofuran (THF) was subsequently added 0.26g of naphthalene and 0.05g of clean sodium particles. After stirring for 30 min. at room temperature the sodium particles were dissolved. To this solution was dropwise added 0.45g of the above prepared p-amino-phenyldiazosulfonate dissolved in 40ml of dry dimethylsulfoxide. After 5 minutes stirring a solution of 0.51g of a copolymer of 40mol% methacryloyl chloride and 60mol% methyl methacrylate in 20ml of dry THF was dropwise added and the obtained reaction mixture was further stirred for 4 hours at room temperature. All the above operations were carried out in the dark avoiding any humidity.

The reaction mixture was then filtered and the copolymer was precipitated in an excess amount of diethylether and subsequently dried. The copolymer was then brought in water free methanol containing 0.03 mol of $NaOCH_3$ to convert the residual acid chloride groups in the copolymer to methyl ester groups. After 2 hours stirring at room temperature the copolymer was precipitated in an excess amount of diethylether and subsequently dried.

A solution of the resulting copolymer was filtered and dialysed for 1 day. The solution was then evaporated under reduced pressure and the resulting copolymer was dried over $P_2O_5$. A copolymer containing 30 mol% of aryldiazosulfonate units and 70 mol% of methyl methacrylate units was thus obtained.

Example 2

Preparation of sodium salt of (4-azosulfonatephenyl)methacrylamide (compound 1 of table 2).

0.O5 mol of p-phenylenediamine and 0.047 mol of triethylamine were dissolved in 80ml of methylene chloride. To this solution was dropwise added while stirring a solution (20ml) of methacryloyl chloride in methylene chloride. The resulting mixture was stirred for 4 days and subsequently 3 times washed with water. The organic phase was dried over $Na_2SO_4$ and the solvent was subsequently removed. The resulting residue was dissolved in a chloroform-acetone mixture and filtered over active carbon black. The product was cristallised at 4 ° C and was further purified by chromatography with silicagel as the solid phase and a mixture of ethylacetate/hexane (2:1) as eluence.

0.00568 mol of the thus prepared 4-aminophenylmethacrylamide was diazotized at -10 ° C with 0.00568 mol $NaNO_2$ in 5ml of a 10% aqueous solution of HCl and subsequently converted with $Na_2SO_3$ dissolved in a solution of sodium acetate at 0 ° C. The resulting sodium salt of (4-azosulfonatephenyl)methacrylamide was recristallized in water.

Preparation of a copolymer of methyl methacrylate and the sodium salt of (4-azosulfonatephenyl)-methacrylamide.

1.45g of the sodium salt of (4-azosulfonatephenyl)methacrylamide and 3.55g of methyl methacrylate and 0.1g of azobisisobutyronitrile were dissolved in a mixture of 40ml water and 10ml dioxane. The solution was then degassed twice with nitrogen. The polymerization was performed at 70 °C in the dark for 10 hours. The polymerization was then ended by adding a little amount of hydroquinone and the solvent was removed by destillation. The resulting residue was dissolved in a methanol dioxane mixture and precipitated in diethyl ether. The obtained polymer was finally dialysed for 24 hours to remove residual sodium salt of (4-azosulfonatephenyl) methacrylamide.

Example 3

To 418 g of a dispersion containing 21.5 % of $TiO_2$ (average particle size 0.3 to 0.5 µm) and 2.5 % polyvinyl alcohol in deionized water were subsequently added, while stirring, 220 g of a 5 % polyvinyl alcohol solution in water, 95 g of a hydrolyzed 22 % tetramethyl orthosilicate emulsion in water and 22 g of a 10 % solution of a wetting agent. To this mixture was then added 245 ml of deionized water and the pH was adjusted to pH = 4.

The resulting dispersion was coated on a polyester support (coated with a hydrophilic adhesion layer) to a wet coating thickness of 55 $g/m^2$ and dried at 30 °C.

On the thus prepared element was coated an aqueous soluton containing 5% of the copolymer prepared as described in example 1 and 0.1% of ULTRAVON W, commercially available from Ciba-Geigy, to a dry thickness of 0.8µm.

The resulting imaging element was exposed through a mask to a high pressure halogen mercury vapour lamp with an emission energy of 800$\mu W/cm^2$ for 2 minutes and subsequently developed with plain water at 25 °C.

A lithographic plate was thus obtained which was used on an AB-Dick 360 printer using a conventional fountain solution and oleophilic lithographic inks.

20000 copies of good quality could be printed.

Example 4

An aluminium foil having a thickness of 0.15 mm was grained, anodized, and sealed according to the method described in Example 1 of US-A 3,861,917. The centre line average value obtained by the graining was 0.5 µm. The thickness of the anodization layer was of 2.7 g per $m^2$.

To the thus obtained aluminium support was coated an aqueous solution containing 5% of the copolymer prepared as described in example 1 and 0.1% of ULTRAVON W, commercially available from Ciba-Geigy, to a dry thickness of 1.1µm. The resulting imaging element was image-wise exposed and developed as described in example 3.

The obtained lithographic printing plate was mounted on an AB-Dick 360 printer using a conventional fountain solution and oleophilic lithographic inks.

35000 copies of good quality could be printed.

Example 5

The same procedure as described in example 4 was followed with the only exception that a copolymer prepared as described in example 2 was used as the light sensitive aryldiazosulfonate coating. 35000 copies of good quality could be printed.

## Claims

**1.** A polymerizable aryldiazosulfonate monomer corresponding to the general formula:

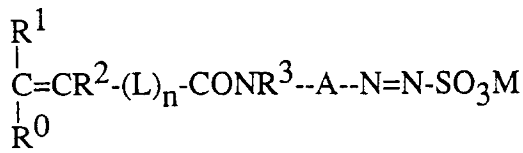

wherein each of $R^0$, $R^1$ and $R^2$ which may be the same or different represents hydrogen or a methyl group, $R^3$ represents hydrogen or an alkyl group, A represents an aromatic divalent group which may be substituted or not, M represents hydrogen, a metal ion or $NH_4^+$, L represents an organic divalent group and n may be 0 or 1.

**2.** A polymerizable aryldiazosulfonate monomer according to claim 1 wherein n of said general formula equals 0 and $R^0$ and $R^1$ both represent hydrogen.

**3.** A polymerizable aryldiazosulfonate monomer according to claim 2 wherein said aryldiazosulfonate monomer is:

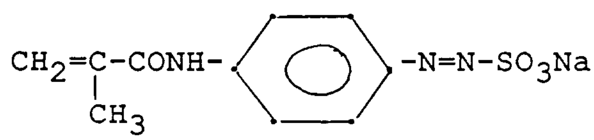

**4.** A homopolymer or copolymer comprising aryldiazosulfonate units corresponding to the general formula:

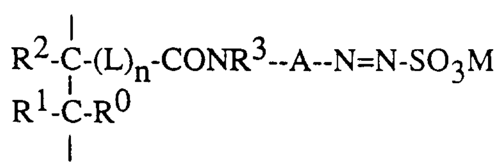

wherein each of $R^0$, $R^1$ and $R^2$, which may be the same or different, represents hydrogen or a methyl group, $R^3$ represents hydrogen or an alkyl group, A represents an aromatic divalent group which may be substituted or not, M represents hydrogen, a metal ion or $NH_4^+$, L represents an organic divalent group and n may be 0 or 1.

**5.** A homopolymer or copolymer according to claim 4 wherein n of said general formula equals 0 and $R^0$ and $R^1$ both represent hydrogen.

**6.** A homopolymer or copolymer according to claim 5 wherein said aryldiazosulfonate unit is

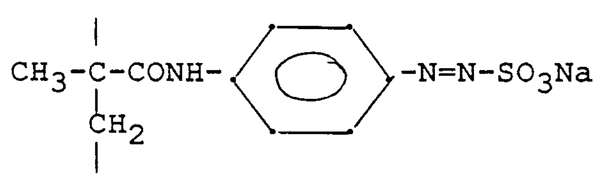

7. An imaging element comprising on a support a polymer comprising aryldiazosulfonate units as defined in any of claims 4 to 6.

8. A method for producing a lithographic printing plate comprising the steps of (i) image-wise exposing an imaging element comprising a homopolymer or copolymer comprising aryldiazosulfonate units as defined in any of claims 4 to 6 and (ii) removing said homopolymer or copolymer in the non-exposed areas.

9. A method according to claim 8 wherein said homopolymer or copolymer is removed by washing said imaging element with water.

## Patentansprüche

1. Ein polymerisierbares Aryldiazosulfonat-Monomeres, das der nachstehenden allgemeinen Formel entspricht:

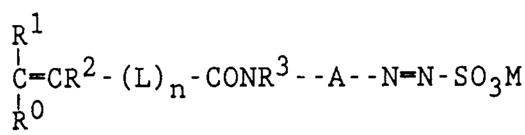

in der $R^0$, $R^1$ und $R^2$, die gleich oder verschieden sein können, je ein Wasserstoffatom oder eine Methylgruppe bedeuten, $R^3$ ein Wasserstoffatom oder eine Alkylgruppe, A eine gegebenenfalls substituierte, aromatische zweiwertige Gruppe, M ein Wasserstoffatom, ein Metallion oder $NH_4^+$ und L eine organische zweiwertige Gruppe bedeuten, und n gleich 0 oder 1 sein kann.

2. Ein polymerisierbares Aryldiazosulfonat-Monomeres nach Anspruch 1, dadurch gekennzeichnet, daß n in der vorstehenden allgemeinen Formel gleich 0 ist und $R^0$ und $R^1$ beide ein Wasserstoffatom bedeuten.

3. Ein polymerisierbares Aryldiazosulfonat-Monomeres nach Anspruch 2, dadurch gekennzeichnet, daß es das folgende ist:

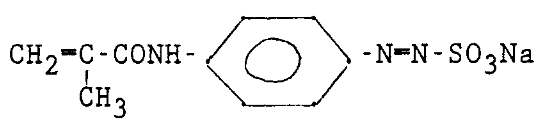

4. Ein Homopolymeres oder Copolymeres, das Aryldiazosulfonat-Einheiten umfaßt, die der nachstehenden allgemeinen Formel entsprechen:

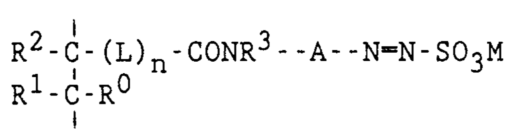

in der $R^0$, $R^1$ und $R^2$, die gleich oder verschieden sein können, je ein Wasserstoffatom oder eine Methylgruppe bedeuten, $R^3$ ein Wasserstoffatom oder eine Alkylgruppe, A eine gegebenenfalls substituierte, aromatische zweiwertige Gruppe, M ein Wasserstoffatom, ein Metallion oder $NH_4^+$, L eine organische zweiwertige Gruppe bedeuten, und n gleich 0 oder 1 sein kann.

5. Ein Homopolymeres oder Copolymeres nach Anspruch 4, dadurch gekennzeichnet, daß n in der vorstehenden allgemeinen Formel gleich 0 ist, und $R^0$ und $R^1$ beide ein Wasserstoffatom bedeuten.

6. Ein Homopolymeres oder Copolymeres nach Anspruch 5, dadurch gekennzeichnet, daß diese Aryldiazosulfonat-Einheit die folgende ist:

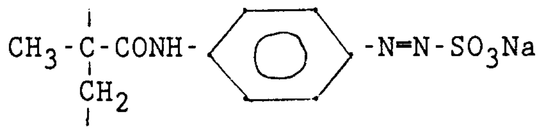

7. Ein bilderzeugendes Element, das auf einem Träger ein Polymeres, das irgendeinem der Ansprüche 4 bis 6 entsprechende Aryldiazosulfonat-Einheiten enthält, umfaßt.

8. Ein Fertigungsverfahren für eine lithografische Druckplatte, das die nachstehenden Schritte umfaßt : (i) das bildmäßige Belichten eines bilderzeugenden Elements, das ein Homopolymeres oder Copolymeres umfaßt, das irgendeinem der Ansprüche 4 bis 6 entsprechende Aryldiazosulfonat-Einheiten enthält, und (ii) das Beseitigen des eingangs genannten Homopolymeren oder Copolymeren in den unbelichteten Bereichen.

9. Ein Verfahren nach Anspruch 8 , dadurch gekennzeichnet, daß dieses Homopolymere oder Copolymere dadurch beseitigt wird, daß dieses bilderzeugende Element mit Wasser gewaschen wird.

**Revendications**

1. Un monomère d'aryldiazosulfonate polymérisable qui répond à la formule générale suivante:

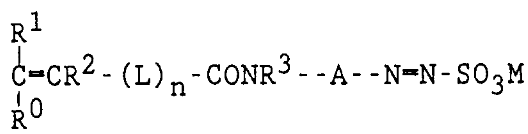

dans laquelle $R^0$, $R^1$ et $R^2$, pouvant être identiques ou différents, représentent chacun un atome d'hydrogène ou un groupe méthyle, $R^3$ représente un atome d'hydrogène ou un groupe alkyle, A représente un groupe aromatique bivalent éventuellement substitué, M représente un atome d'hydrogène, un ion métal ou $NH_4^+$, L représente un groupe organique bivalent et n peut être égal à 0 ou 1.

2. Un monomère d'aryldiazosulfonate polymérisable suivant la revendication 1, caractérisé en ce que n dans la formule générale précitée est égal à 0, et $R^0$ et $R^1$ représentent tous les deux un atome d'hydrogène.

3. Un monomère d'aryldiazosulfonate polymérisable suivant la revendication 2, caractérisé en ce qu'il est le suivant:

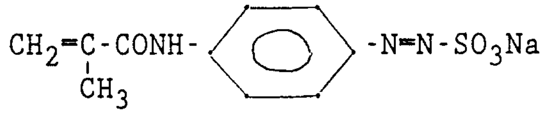

4. Un homopolymère ou copolymère comprenant des unités d'aryldiazosulfonate qui répondent à la formule générale suivante:

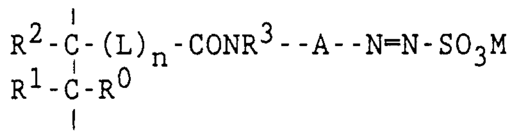

dans laquelle $R^0$, $R^1$ et $R^2$, pouvant être identiques ou différents, représentent chacun un atome d'hydrogène ou un groupe méthyle, $R^3$ représente un atome d'hydrogène ou un groupe alkyle, A représente un groupe aromatique bivalent éventuellement substitué, M représente un atome d'hydrogène, un ion métal ou $NH_4^+$, L représente un groupe organique bivalent, et n peut être égal à 0 ou 1.

5. Un homopolymère ou copolymère suivant la revendication 4, caractérisé en ce que n dans la formule générale précitée est égal à 0, et $R^0$ et $R^1$ représentent tous les deux un atome d'hydrogène.

6. Un homopolymère ou copolymère suivant la revendication 5, caractérisé en ce que l'unité d'aryldiazosulfonate précité est la suivante:

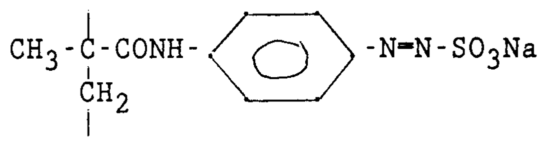

7. Un élément formateur d'image comprenant sur un support un polymère renfermant des unités d'aryldiazosulfonate conformes à l'une quelconque des revendications 4 à 6.

8. Un procédé pour la confection d'une plaque d'impression lithographique comportant les opérations (i) d'exposition sous forme d'image d'un élément formateur d'image comprenant un homopolymère ou copolymère renfermant des unités d'aryldiazosulfonate conformes à l'une quelconque des revendications 4 à 6 et (ii) d'enlèvement de l'homopolymère ou du copolymère précité dans les zones non-exposées.

9. Un procédé suivant la revendication 8, caractérisé en ce que l'homopolymère ou le copolymère précité est enlevé par lavage à l'eau de l'élément formateur d'image précité.